# EUROPÄISCHE PATENTSCHRIFT

(11) **EP 1 601 638 B1**
(45) Veröffentlichungstag und Bekanntmachung des Hinweises auf die Patenterteilung: **27.12.2006**
(21) Anmeldenummer: 04714752.5
(22) Anmeldetag: 26.02.2004
(51) Int. Cl.: C07C 45/78, C07C 45/48, C07C 49/395, B01J 23/04

(54) **VERFAHREN ZUR HERSTELLUNG UND REINIGUNG VON CYCLOPENTANON**
METHOD FOR PRODUCING AND PURIFYING CYCLOPENTANONE
PROCEDE DE FABRICATION ET D'EPURATION DE CYCLOPENTANONE

(30) Priorität: 26.02.2003 DE 10308488
(43) Veröffentlichungstag der Anmeldung: 07.12.2005
(73) Patentinhaber: BASF Aktiengesellschaft, 67056 Ludwigshafen (DE)
(72) Erfinder: FISCHER, Rolf-Hartmuth, 69121 Heidelberg (DE); GALL, Martin, 67112 Mutterstadt (DE); HUBER-DIRR, Sylvia, 64673 Zwingenberg (DE); LIANG, Shelue, 67071 Ludwigshafen (DE); NÖBEL, Thomas, 67133 Maxdorf (DE); RUST, Harald, 67435 Neustadt (DE); STEIN, Frank, 67098 Bad Dürkheim (DE)
(74) Vertreter: Isenbruck, Günter
(86) Internationale Anmeldenummer: PCT/EP2004/001909
(87) Internationale Veröffentlichungsnummer: WO 2004/076396

(56) Entgegenhaltungen:
- WO-A-99/61402
- US-A- 2 513 534

## Beschreibung

Die vorliegende Erfindung betrifft ein Verfahren zur Herstellung von hochreinem Cyclopentanon sowie die Verwendung von Alkalioxiden auf oxidischen Trägern zur Herstellung von Cyclopentanon.

Cyclopentanon ist ein wichtiges Zwischenprodukt in Synthesen von verschiedenen chemischen Produkten. Stellvertretend wird diesbezüglich auf die Verwendung von Cyclopentanon zur Herstellung von Methyldihydrojasmonat (Hedion) verwiesen, welches auf Grund seines blumig-jasminartigen Duftes in zahlreichen Parfüms bzw. Duftzusammensetzungen verwendet wird.

Aus dem Stand der Technik sind zahlreiche Verfahren zur Herstellung von Cyclopentanon bekannt.

So beschreibt beispielsweise die DE-A 3 730 185 die Herstellung von Cyclopentanon ausgehend von Adipinsäure in der Gasphase bei Temperaturen von 250 bis 450 °C. Die Herstellung von Cyclopentanon erfolgt dabei gegebenenfalls in Gegenwart von Wasserdampf über oxidischen sauren oder basischen Katalysatoren, bevorzugt auf Wirbelbett-Katalysatoren. Beispiele für geeignete Katalysatoren sind mit Elementen der I. oder II. Hauptgruppe dotierte Aluminiumoxide oder Phosphorsäure bzw. Hydrogenphosphate enthaltene Kieselsäuren. Die Reinheit des so erhaltenen Cyclopentanons ist nicht angegeben.

Aus der EP-A 0 626 363 ist bekannt, dass Adipinsäure in flüssiger Phase bei Temperaturen von 200 bis 300 °C in Gegenwart eines Metalls oder einer Metallverbindung des Bors, Aluminiums, Galliums, Iridiums, Thalliums, Zinns, Antimons, Bismuths, Molybdäns, Rubidiums, Cäsiums oder Vanadiums zu Cyclopentanon umgesetzt werden kann. Das Cyclopentanon wird abschließend durch Kristallisation gereinigt.

Die EP-A 0 251 111 beschreibt die Cyclisierung von aliphatischen Dicarbonsäureestern mit 6 bis 8 Kohlenstoffatomen, beispielsweise Adipinsäurediestem, in der Gas- oder Flüssigphase bei Temperaturen von 300 bis 345 °C zu Cyclopentanon. Dabei wird an festen oxidischen Katalysatoren gearbeitet, denen Elemente der I. bis V. Hauptgruppe des Periodensystems der Elemente, der Seltenerdmetalle oder Gemische davon zugrunde liegen. In einer bevorzugten Ausführungsform wird bei der Cyclisierung Wasser zugesetzt, um die Selektivität und Standzeit der verwendeten Katalysatoren zu erhöhen. Die Reinheit des so erhaltenen Cyclopentanons ist nicht angegeben.

Als Katalysatoren können nach der EP- A 1 084 096 auch Titandioxid, Zirkondioxid oder seltene Erdoxide eingesetzt werden, die Metalloxide der I. bzw. II. Hauptgruppe des Periodensystems der Elemente enthalten. Darüber hinaus können Erdalkalioxide auf Zinkoxid verwendet werden. Die Reinheit des so erhaltenen Cyclopentanons ist nicht angegeben.

Weiterhin lassen sich nach der EP-A 1 015 408 5-Formylvaleriansäure und ihre Ester oder 6-Hydroxycapronsäure und ihre Ester bei Temperaturen von 200 bis 450 °C in der Gas- oder Flüssigphase an oxidischen Katalysatoren zu Cyclopentanon umsetzten. Auch hier wird in einer bevorzugten Ausführungsform zur Erhöhung der Selektivität und Standzeit der Katalysatoren Wasser zugesetzt. Die Reinheit des so erhaltenen Cyclopentanons ist nicht angegeben.

WO 99/61402 beschreibt ebenfalls die Herstellung von Cyclopentanon.

Bei der Cyclisierung von Adipinsäure, 5-Formylvaleriansäure und 6-Hydroxycapronsäure entsteht neben Cyclopentanon je ein Mol Wasser. Verwendet man Adipinsäuremonoester, Adipinsäurediester und/oder 6-Hydroxycapronsäuremethylester, so findet keine Wasserbildung statt. In bevorzugten Ausführungsformen dieser Cyclisierungen wird dem Reaktionsgemisch jedoch Wasser zugesetzt.

Da Cyclopentanon mit Wasser ein Heteroazeotrop bildet (Siedepunkt 93°C/1013 mbar), ist eine einfache destillative Abtrennung des Wassers vom Cyclopentanon nicht möglich.

Bislang praktizierte Verfahrensschritte zur Aufreinigung von Cyclopentanon sind Phasentrennung, Reinigung durch Kristallisation des Cyclopentanons oder Vergrößerung der Mischungslücke Cyclopentanon/Wasser durch Aussalzen mit Natriumchlorid. Alle diese beschriebenen Maßnahmen erfolgen zusätzlich zu einer zwingend notwendigen Destillation der aus den jeweiligen Verfahren gewonnenen Cyclopentanon/Wasser-Gemische, um hochreines Cyclopentanon zu erhalten. Ein kontinuierliches Verfahren zur rein destillativen Auftrennung der Cyclopentanon/Wasser-Gemische, wobei hochreines Cyclopentanon unter Vermeidung weiterer zusätzlicher Reinigungsschritte erhalten wird, ist im oben genannten Stand der Technik nicht beschrieben.

Es bestand daher die Aufgabe, ein Verfahren zur Herstellung von Cyclopentanon bereit zu stellen, bei dem die Cyclisierung mit hoher Ausbeute und Standzeit des Cyclisierungskatalysators verläuft und sich das dabei anfallende Cyclopentanon/Wasser-Gemisch mit möglichst geringem apparativem und energetischem Aufwand zu Cyclopentanon aufarbeiten lässt. Dabei sollte das Verfahren so flexibel sein, dass alle aus Adipinsäure und ihren Derivaten hervorgehenden Cyclopentanon/Wasser-Gemische, die gegebenenfalls zusätzlich noch niedere Alkohole, beispielsweise Methanol, enthalten, aufgereinigt werden können.

Es wurde erfindungsgemäß gefunden, dass die vorliegende Aufgabe durch das im folgenden beschriebene Verfahren gemäß Ausführungsform I und Ausführungsform II gelöst wird.

Gegenstand der vorliegenden Erfindung ist somit gemäß **Ausführungsform I** ein kontinuierliches Verfahren zur Herstellung von Cyclopentanon, das die folgenden Verfahrensschritte umfasst:
(a) Bereitstellung einer Mischung, die erhalten wird
   durch Umsetzung
   mindestens einer Verbindung der allgemeinen Formel (I)

   X-(CH₂)₄-COOR (I),

   in der X einen Carbonsäure-, Carbonsäureester-, Formyl- oder Hydroxymethylrest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder
   durch Umsetzung
   mindestens einer Verbindung, die mit Wasser und/oder Alkoholen in mindestens eine Verbindung der allgemeinen Formel (I) übergehen kann, an
   mindestens einem Katalysator gegebenenfalls in Gegenwart von Wasser,
(b) Überführen der in Verfahrensschritt (a) erhaltenen Mischung enthaltend Cyclopentanon, Wasser und gegebenenfalls Hoch- und/oder Leichtsieder in eine erste Kolonne, worin eine Trennung der Mischung in ein Cyclopentanon/Wasser-Gemisch und gegebenenfalls vorhandene Hoch- und/oder Leichtsieder erfolgt, und Ausschleusen des Cyclopentanon/Wasser-Gemisches durch einen Seitenabzug der ersten Kolonne oder als Kopfprodukt der ersten Kolonne, wobei das Cyclopentanon/Wasser-Gemisch gegebenenfalls noch geringe Mengen an Hoch- und/oder Leichtsieder enthält,
(c) Trennung des in Verfahrensschritt (b) erhaltenen Cyclopentanon/Wasser-Gemisches in eine wässrige Phase enthaltend Wasser und gegebenenfalls geringe Mengen Cyclopentanon und eine organische Phase enthaltend Cyclopentanon und gegebenenfalls geringe Mengen Wasser in einem Phasenscheider, wobei die organische und/oder wässrige Phase gegebenenfalls noch geringe Mengen Hoch- und/oder Leichtsieder enthält,
(d) Überführen der in Verfahrensschritt (c) erhaltenen wässrigen Phase in eine zweite Kolonne, worin eine Trennung der wässrigen Phase in Wasser, ein Cyclopentanon/Wasser-Gemisch und gegebenenfalls noch vorhandene, in dem Verfahrensschritt (b) nicht abgetrennte und/oder zwischenzeitlich neu gebildete Hoch- und/oder Leichtsieder erfolgt, und Ausschleusen von Leichtsiedern über den Kopf der zweiten Kolonne, von Wasser und gegebenenfalls Hochsiedem über den Sumpf der zweiten Kolonne und von einem Cyclopentanon/Wasser-Gemisch über einen Seitenabzug der zweiten Kolonne, wobei das Cyclopentanon/Wasser-Gemisch gegebenenfalls in den Phasenscheider des Verfahrensschrittes (c) zurückgeführt wird,
(e) Überführen der in Verfahrensschritt (c) erhaltenen organischen Phase enthaltend Cyclopentanon in eine dritte Kolonne, worin eine Trennung der organischen Phase in gegebenenfalls noch vorhandene, in dem Verfahrensschritt (b) nicht abgetrennte und/oder zwischenzeitlich neu gebildete Leicht- und/oder Hochsieder und Cyclopentanon erfolgt, Ausschleusen der über den Kopf der dritten Kolonne gewonnenen Leichtsieder sowie der über Sumpf gewonnenen Hochsieder, Ausschleusen und gegebenenfalls Zurückführen des ebenfalls über Kopf der dritten Kolonne gewonnenen Cyclopentanon/Wasser-Gemisches in den Phasenscheider des Verfahrensschrittes (c) und Erhalt des Cyclopentanons im Seitenabzug der dritten Kolonne.

Weiterer Gegenstand der vorliegenden Erfindung ist ein kontinuierliches Verfahren zur Herstellung von Cyclopentanon gemäß Ausführungsform II, das die folgenden Verfahrensschritte umfasst:
(a) Bereitstellung einer Mischung, die erhalten wird
   durch Umsetzung
   mindestens einer Verbindung der allgemeinen Formel (I)

   X-(CH₂)₄-COOR (I),

   in der X einen Carbonsäure-, Carbonsäureester-, Formyl- oder Hydroxymethylrest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder
   durch Umsetzung
   mindestens einer Verbindung, die mit Wasser und/oder Alkoholen in mindestens eine Verbindung der allgemeinen Formel (I) übergehen kann, an
   mindestens einem Katalysator gegebenenfalls in Gegenwart von Wasser,
(b) Überführen der in Verfahrensschritt (a) erhaltenen Mischung enthaltend Cyclopentanon, Wasser und gegebenenfalls Hoch- und/oder Leichtsieder in eine erste Kolonne, worin eine Trennung der Mischung in ein Cyclopentanon/Wasser-Gemisch und gegebenenfalls vorhandene Hoch- und/oder Leichtsieder erfolgt, und Ausschleusen des Cyclopentanon/Wasser-Gemisches durch einen Seitenabzug der ersten Kolonne bei Anwesenheit von Leichtsiedern oder als Kopfprodukt der ersten Kolonne bei Abwesenheit von Leichtsiedern, wobei das Cyclopentanon/Wasser-Gemisch gegebenenfalls noch geringe Mengen an Hoch- und/oder Leichtsieder enthält,
(c) Trennung des in Verfahrensschritt (b) erhaltenen Cyclopentanon/Wasser-Gemisches in eine wässrige Phase enthaltend Wasser und gegebenenfalls geringe Mengen Cyclopentanon und eine organische Phase enthaltend Cyclopentanon und gegebenenfalls geringe Mengen an Wasser in einem Phasenscheider, wobei die wässrige Phase gegebenenfalls in die erste Kolonne des Verfahrensschrittes (b) zurückgeführt wird und die organische und/oder wässrige Phase gegebenenfalls noch geringe Mengen Hoch- und/oder Leichtsieder enthält,
(d) Überführen der in Verfahrensschritt (c) erhaltenen organischen Phase in eine Trennwandkolonne, worin eine Trennung der organischen Phase in gegebenenfalls noch vorhandene, in dem Verfahrensschritt (b) nicht abgetrennte und/oder zwischenzeitlich neu gebildete Leicht- und/oder Hochsieder, ein Cyclopentanon/Wasser-Gemisch und Cyclopentanon erfolgt, und Ausschleusen von Leichtsiedern und dem Cyclopentanon/Wasser-Gemisch über den Kopf der Trennwandkolonne, von Hochsiedem über den Sumpf der Trennwandkolonne und von Cyclopentanon über einen Seitenabzug der Trennwandkolonne, wobei das Cyclopentanon/Wasser-Gemisch gegebenenfalls in den Phasenscheider des Verfahrensschrittes (c) zurückgeführt wird.

Unter einem Phasenscheider wird im Sinne der vorliegenden Erfindung jede Apparatur oder Anordnung verstanden, mit der zwei flüssige Phasen voneinander getrennt werden können.

Durch das erfindungsgemäße Verfahren gemäß Ausführungsform I und II wird hochreines Cyclopentanon erhalten, welches eine Reinheit von im Allgemeinen mindestens 99 %, bevorzugt mindestens 99,5 %, besonders bevorzugt mindestens 99,9 %, aufweist. Der Wassergehalt beträgt im Allgemeinen weniger als 2.500 ppm, bevorzugt weniger als 2.000 ppm, besonders bevorzugt weniger als 1.500 ppm. Das Cyclopentanon weist im Allgemeinen eine Farbzahl von weniger als 20 APHA, bevorzugt weniger als 15 APHA, besonders bevorzugt weniger als 10 APHA, auf.

Im Folgenden werden unter der Bezeichnung "Verbindung der allgemeinen Formel (I)" die Verbindungen der allgemeinen Formel (I) und die Verbindungen, die mit Wasser und/oder Alkoholen in mindestens eine Verbindung der allgemeinen Formel (I) übergehen, verstanden.

Unter den Begriffen "über den Kopf der (Trennwand-)Kolonne" bzw. "als Kopfprodukt" werden im Sinne der vorliegenden Erfindung eine Entnahmestelle der betreffenden Kolonne bzw. das Produkt dieser Entnahmestelle verstanden, wobei sich die Entnahmestelle im Allgemeinen auf mindestens 75 % der Gesamthöhe der jeweiligen Kolonne, bevorzugt mindestens 80 % der Gesamthöhe der jeweiligen Kolonne, besonders bevorzugt mindestens 85 % der Gesamthöhe der jeweiligen Kolonne, jeweils ausgehend von dem Boden der Kolonne, befindet. Somit wird erfindungsgemäß unter dem Begriff "über den Kopf der (Trennwand-)Kolonne" auch eine hohe seitliche Entnahmestelle verstanden.

### Ausführungsform I

### Verfahrensschritt (a)

Das erfindungsgemäße kontinuierliche Verfahren zur Herstellung von Cyclopentanon gemäß Ausführungsform I umfasst zunächst den Verfahrensschritt (a), bei dem eine Mischung bereitgestellt wird, die erhalten wird durch Umsetzung mindestens einer Verbindung der allgemeinen Formel (I)

X-(CH₂)₄-COOR (I),

in der X einen Carbonsäure-, Carbonsäureester-, Formyl- oder Hydroxymethyl-Rest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt,
und/oder durch Umsetzung mindestens einer Verbindung, die mit Wasser und/oder Alkohol in mindestens eine Verbindung der allgemeinen Formel (I) übergehen kann, an mindestens einem Katalysator, wobei die Umsetzung gegebenenfalls in Gegenwart von Wasser erfolgt.

Beispiele für als Ausgangsstoffe eingesetzte Verbindungen der allgemeinen Formel (I) sind Adipinsäure, 5-Formylvaleriansäure und 6-Hydroxycapronsäure und ihre Ester, wobei die Ester aliphatische Reste mit 1 bis 6 Kohlenstoffatomen oder cycloaliphatische, aromatische oder araliphatische Reste mit 5 bis 12, vorzugsweise 6 bis 8 Kohlenstoffatomen, enthalten können. Beispiele für diese Reste R sind Methyl-, Ethyl-, Propyl-, Isopropyl-, Butyl-, Isobutyl-, tert.-Butyl-, Hexyl-, Cyclopentyl-, Cyclohexyl-, Phenyl- oder Benzylreste.

Unter einer Verbindung, die mit Wasser und/oder Alkoholen in mindestens eine Verbindung der allgemeinen Formel (I) übergehen kann, werden im Sinne der vorliegenden Erfindung alle Verbindungen verstanden, die unter den angegebenen Reaktionsbedingungen des Verfahrenschrittes (a) Verbindungen der allgemeinen Formel (I) bilden. So kann man anstelle von 6-Hydroxycapronsäure oder 6-Hydroxycapronsäureestern Gemische von Caprolacton mit Wasser oder Alkoholen verwenden. Weitere Beispiele für Verbindungen, die mit Wasser und/oder Alkoholen in mindestens eine Verbindung der allgemeinen Formel (I) übergehen können, sind Carbonsäureamide und Carbonsäurechloride der allgemeinen Formel (I), worin R = NR* oder R = Cl ist, wobei die Amidfunktion NR* beliebig sein kann mit der Maßgabe, dass das Carbonsäureamid unter den Bedingungen des Verfahrensschrittes (a) in eine Verbindung der allgemeinen Formel (I) übergeht. Das für diese Umsetzung notwendige Wasser bzw. der für diese Umsetzung notwendige Alkohol wird dem erfindungsgemäßen Verfahren gemäß Ausführungsform I oder II gegebenenfalls zugesetzt.

Als Katalysatoren in Verfahrensschritt (a) sind Metalle, Metalloxide, Metallhydroxide, Metallsalze und/oder Metall-Doppelsalze von Elementen der I. bis XIV. Gruppe des Periodensystems der Elemente und/oder der Seltenerdmetalle geeignet. Als Oxide bzw. Hydroxide sind beispielsweise
- Alkalioxide wie Natriumoxid, Kaliumoxid,
- Erdalkalioxide wie Magnesiumoxid, Calciumoxid, Bariumoxid,
- Bortrioxid, Aluminiumoxide,
- Siliziumdioxide, z. B. in Form von Kieselgel, Kieselgur, Silikaten oder Quarz,
- Zinndioxid, Bismutoxid, Kupferoxide, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide,
- Natriumhydroxid, Kaliumhydroxid, Calciumhydroxid,
- oder Gemische derartiger Oxide und Hydroxide
geeignet.

Als Metallsalze und Metall-Doppelsalze sind beispielsweise Acetate, Phosphate, Borate, Carbonate, Vanadate, Oxalate, Silikate, Sulfate und Adipate geeignet. Beispiele hierfür sind Kaliumcarbonat, Cäsiumcarbonat, Natriumcarbonat, Natriumacetat, Lithiumphosphate, Natriumphosphate, Kaliumphosphate, Natriumborate, Kaliumvanadate, Aluminiumphosphate, Cerphosphate, Borphosphate, Eisenaluminiumphosphate, Zirkonphosphate, Calciumphosphate, Siliziumaluminiumphosphate, Zeolithe, Mono- und Dinatriumadipat.

Besonders bevorzugt sind feste, oxidische Katalysatoren, welche auf einem oxidischen Trägermaterial im Allgemeinen 0,01 bis 5 Gew.-%, bevorzugt 0,1 bis 3 Gew.-%, besonders bevorzugt 0,3 bis 2 Gew.-% eines Alkalioxids und/oder Seltenerdoxids, jeweils bezogen auf den gesamten Katalysator, aufweisen.

Als Alkalioxide kommen beispielsweise Lithium-, Natrium-, Kalium-, Rubidium- und Cäsiumoxide oder Gemische dieser Oxide in Frage. Besonders bevorzugt sind Natrium- und Kaliumoxid als katalytisch aktive Materialien. Als Seltenerdoxide kommen z. B. Lanthanoxide in Frage.

Als Trägermaterial für die Katalysatoren in Verfahrensschritt (a) des erfindungsgemäßen Verfahrens gemäß Ausführungsform I werden vorzugsweise Metalloxide der II. bis V. Hauptgruppe, der I. bis VIII. Nebengruppe des Periodensystems der Elemente oder Oxide der Seltenerdmetalle oder Gemische dieser Oxide verwendet. Beispiele für derartige Träger sind Magnesiumoxid, Calciumoxid, Bariumoxide, Bortrioxid, Aluminiumoxide, Siliziumoxide, beispielsweise in Form von Kieselgel, Kieselgur oder Quarz, Zinndioxid, Bismutoxide, Kupferoxide, Zinkoxid, Lanthanoxid, Titanoxide, Zirkondioxid, Vanadiumoxide, Chromoxide, Molybdänoxide, Wolframoxide, Manganoxide, Eisenoxide, Ceroxide, Neodymoxide oder Gemische derartiger Oxide. Besonders bevorzugt wird das Trägermaterial ausgewählt aus der Gruppe bestehend aus Aluminiumoxiden, Siliziumdioxid, Titandioxid und deren Gemischen.

Bevorzugt werden als Trägermaterial Aluminiumoxide, Zinkoxid, Zirkondioxid, Titanoxide und/oder Siliziumoxide verwendet, ganz besonders bevorzugt sind Aluminiumoxide und/oder Siliziumoxide.

Für das erfindungsgemäße Verfahren eignen sich somit beispielsweise La₂O₃, ZrO₂, Cr₂O₃/ZrO₂, CaO/ZnO, MgO/ZnO, K₂O/TiO₂, La₂O₃/Al₂O₃, ZrOSO₄, K₂O/SiO₂, Na₂O/Al₂O₃, Na₂O/SiO₂, Na₂O/Al₂O₃ und K₂O/Al₂O₃.

Erfindungsgemäß ist es auch möglich, unterschiedliche Katalysatoren gleichzeitig und/oder hintereinander zu verwenden.

Die im erfindungsgemäßen Verfahren gemäß Ausführungsform I in Verfahrensschritt (a) verwendeten Katalysatoren können in allen beliebigen Darreichungsformen verwendet werden, beispielsweise als Pulver, als 2- bis 5-mm-Stränge, als Tabletten mit beispielsweise 3 bis 5 mm Durchmesser, als Split mit Teilchengrößen von beispielsweise 0,1 bis 0,5 mm oder als Wirbelkontakt.

Die erfindungsgemäß bevorzugt eingesetzten Trägerkatalysatoren können nach an sich bekannten Verfahren, beispielsweise durch Fällung der katalytisch aktiven Komponente aus ihren Salzlösungen in Gegenwart des Trägermaterials oder durch Zugabe von Alkalimetallhydroxid- oder -carbonat-Lösungen, hergestellt werden. Die jeweiligen Hydroxide, Oxidhydrate, basischen Salze oder Carbonate werden so ausgefällt.
Die Niederschläge werden anschließend getrocknet und durch Calcinierung bei im Allgemeinen 300 bis 1300 °C, bevorzugt 400 bis 1200 °C, in die entsprechenden Oxide, Mischoxide und/oder gemischtvalentigen Oxide umgewandelt.

Neben den oben genannten Fällungskatalysatoren, die als Trägerkatalysatoren eingesetzt werden können, sind auch Trägerkatalysatoren geeignet, bei denen die katalytisch aktiven Komponenten andersartig auf das Trägermaterial aufgebracht worden sind.

Beispielsweise können die katalytisch aktiven Komponenten durch Tränkung mit Lösungen oder Suspensionen der Salze oder Oxide der entsprechenden Elemente und Trocknung aufgebracht werden.

Die katalytisch aktiven Komponenten können auch auf den Träger aufgebracht werden, indem der Träger mit Lösungen thermisch leicht zersetzbarer Salze imprägniert wird und der so behandelte Träger auf Temperaturen von im Allgemeinen 300 bis 600 °C erhitzt wird, wobei die adsorbierten Metallverbindungen thermisch zersetzt werden.

Thermisch leicht zersetzbare Salze sind beispielsweise Nitrate und Komplexverbindungen, wie Carbonyl- oder Hydrido-Komplexe, der katalytisch aktiven Metalle. Die thermische Zersetzung wird vorzugsweise in einer Schutzgasatmosphäre durchgeführt. Geeignete Schutzgase sind beispielsweise Stickstoff, Kohlendioxid, Wasserstoff oder Edelgase.

Weiterhin kann die katalytisch aktive Komponente durch Aufdampfen oder Flammspritzen auf dem Trägermaterial abgeschieden werden.

Die Umsetzung (Verfahrensschritt (a)) findet im Allgemeinen bei Temperaturen von 200 bis 400 °C, bevorzugt bei 210 bis 350 °C, besonders bevorzugt bei 220 bis 330 °C, statt. Im Allgemeinen wird die Reaktion unter Atmosphärendruck durchgeführt. Gleichfalls ist es möglich, schwach verminderten oder schwach erhöhten Druck, beispielsweise zwischen 20 mbar und 5 bar, anzuwenden. Die Katalysatorbelastung liegt im Allgemeinen bei 0,1 bis 4 kg wasserfreie Verbindung der allgemeinen Formel (I) pro kg Katalysator und pro Stunde, bevorzugt 0,1 bis 2 kg wasserfreie Verbindung der allgemeinen Formel (I) pro kg Katalysator und pro Stunde, besonders bevorzugt 0,1 bis 1 kg wasserfreie Verbindung der allgemeinen Formel (I) pro kg Katalysator und pro Stunde.

Die Umsetzung (Verfahrensschritt (a)) der Verbindung der allgemeinen Formel (I) an einem der genannten Katalysatoren erfolgt in der Gas- oder Flüssigphase, gegebenenfalls unter Mitverwendung von Verdünnungsmitteln. Beispiele für Verdünnungsmittel sind Lösemittel, die unter den angegebenen Reaktionsbedingungen vollständig oder weitgehend vollständig inert sind, beispielsweise Ether (Dioxan oder Tetrahydrofuran, Biphenylether, Dibenzylether) oder Kohlenwasserstoffe wie Dodekan oder Weißöle.

Die Umsetzung (Verfahrensschritt (a)) kann diskontinuierlich oder kontinuierlich, vorzugsweise als Festbettreaktion mit Festbettkatalysatoren, beispielsweise in Sumpf- oder Rieselfahrweise, in der Gas- oder Flüssigphase, als Wirbelbettreaktion mit in auf- und abwirbelnder Bewegung befindlichen Katalysatoren oder aber mit in der Flüssigphase gelösten oder suspendierten Katalysatoren durchgeführt werden. Bevorzugt erfolgt die Umsetzung kontinuierlich.

Bei Verwendung eines Wirbelbetts kann die Ausgangsverbindung der allgemeinen Formel (I) in gasförmiger, fester oder flüssiger Form in das Wirbelbett eingebracht werden.

Die Umsetzung (Verfahrensschritt (a)) in der Flüssigphase wird beispielsweise so durchgeführt, dass man Ausgangsverbindungen der allgemeinen Formel (I), gegebenenfalls in Anwesenheit eines Verdünnungs- bzw. Lösemittels, in Gegenwart eines gelösten, suspendierten oder festangeordneten Katalysators auf die gewünschte Reaktionstemperatur erhitzt und das entstehende Cyclopentanon durch Extraktion oder Destillation aus dem Reaktionsgemisch gewonnen wird. In einer bevorzugten Ausführungsform wird das Cyclopentanon zusammen mit Wasser kontinuierlich aus dem Reaktionsgemisch abdestilliert.

Suspendierte Katalysatoren können bei diskontinuierlicher Fahrweise nach der Gewinnung von Cyclopentanon durch Dekantieren, Filtrieren oder Zentrifugieren abgetrennt werden. Gelöste Katalysatoren verbleiben im Allgemeinen im Destillationsrückstand.

Es ist zwar möglich, den Verfahrensschritt (a) ohne Zusatz von Wasser durchzuführen, allerdings wird durch die Zugabe von Wasser eine bemerkenswerte Erhöhung der Selektivität und Standzeit erreicht. Das Molverhältnis von Ausgangsstoff der allgemeinen Formel (I) zu Wasser beträgt hierbei im Allgemeinen 1:0,01 bis 1:50, bevorzugt 1:0,01 bis 1:20, besonders bevorzugt 1:0,5 bis 1:10.

Die bei der Cyclisierung der Ausgangsverbindungen der allgemeinen Formel (I) an einem erfindungsgemäßen Katalysator entstehenden Reaktionsgemische enthalten u. a. Cyclopentanon, Wasser, gegebenenfalls Leichtsieder, beispielsweise niedere Alkohole, Hochsieder, beispielsweise Cyclopentanon-2-carbonsäureester, durch Aldolreaktion dimerisiertes Cyclopentanon, und bei nicht vollständigem Umsatz Ausgangsverbindungen der allgemeinen Formel (I).

### Verfahrensschritt (b)

Der Verfahrensschrift (b) des erfindungsgemäßen Verfahrens gemäß Ausführungsform I umfasst das Überführen der in Verfahrensschritt (a) erhaltenen Mischung aus Cyclopentanon, Wasser und gegebenenfalls Hoch- und/oder Leichtsiedern in eine erste Kolonne K1, worin eine Trennung der Mischung in ein Cyclopentanon/Wasser-Gemisch und gegebenenfalls vorhandene Hoch- und/oder Leichtsieder erfolgt, gefolgt von einem Ausschleusen des Cyclopentanon/Wasser-Gemisches durch einen Seitenabzug der ersten Kolonne bei Anwesenheit von Leichtsiedern oder als Kopfprodukt der ersten Kolonne bei Abwesenheit von Leichtsiedern.

Für die Destillation kommen hierbei übliche Apparaturen in Betracht, wie sie beispielsweise in Kirk-Othmer, Encyclopedia of Chemical Technology, 3. Ed., Vol. 7, John Wiley & Sons, New York, 1979, Seiten 870-881 beschrieben sind, beispielsweise Siebbodenkolonnen, Glockenbodenkolonnen, Packungskolonnen oder Füllkörperkolonnen.

Die Trennung der aus Verfahrensschritt (a) erhaltenen Mischung aus Cyclopentanon, Wasser und gegebenenfalls Hoch- und/oder Leichtsiedern erfolgt nicht zwingend vollständig, so dass das aus dieser Destillation resultierende Cyclopentanon/Wasser-Gemisch gegebenenfalls noch geringe Mengen Hoch- und/oder Leichtsieder enthält.

Die in Verfahrensschritt (b) verwendete erste Kolonne K1 weist im Allgemeinen 40 bis 80 theoretische Böden, bevorzugt 50 bis 70 theoretische Böden, besonders bevorzugt 55 bis 65 theoretische Böden auf. Die Kolonne K1 wird im Allgemeinen bei einer Sumpftemperatur von 70 bis 120 °C, bevorzugt 80 bis 110 °C, besonders bevorzugt 90 bis 100 °C, betrieben.

Der Druck in der ersten Kolonne in Verfahrensschritt (b) beträgt im Allgemeinen 800 bis 1000 mbar, bevorzugt 850 bis 950 mbar, besonders bevorzugt 875 bis 925 mbar.

### Verfahrensschritt (c)

Das im Verfahrensschritt (b) des erfindungsgemäßen Verfahrens gemäß Ausführungsform I erhaltene gasförmige Cyclopentanon/Wasser-Gemisch wird in eine wässrige Phase und eine organische Phase getrennt. Diese Trennung erfolgt im Allgemeinen durch Kondensation des Cyclopentanon/Wasser-Gemisches auf Temperaturen, bei denen sich zwei Phasen bilden, beispielsweise 40 °C. Die Trennung in Verfahrensschritt (c) erfolgt dabei in einem Phasenscheider. Die so gebildete wässrige Phase kann gegebenenfalls noch geringe Mengen an Cyclopentanon enthalten, während die organische Phase Cyclopentanon und darüber hinaus gegebenenfalls geringe Mengen Wasser enthält.

Die organische Phase enthält im Allgemeinen 75 bis 90 Gew.-%, bevorzugt 77,5 bis 87,5 Gew.-%, besonders bevorzugt 80 bis 85 Gew.-% Cyclopentanon, jeweils bezogen auf die organische Phase. Die wässrige Phase enthält im Allgemeinen 35 bis 15 Gew.-%, bevorzugt 32,5 bis 17,5 Gew.-%, besonders bevorzugt 30 bis 20 Gew.-%, Cyclopentanon, jeweils bezogen auf die wässrige Phase.

Die in Verfahrensschritt (c) erzeugte organische und/oder wässrige Phase kann gegebenenfalls noch geringe Mengen Hoch- und/oder Leichtsieder enthalten, die entweder im Verfahrensschritt (b) nicht vollständig entfernt wurden, und/oder zwischenzeitlich aus dem Cyclopentanon und den weiteren gegebenenfalls vorhandenen Verbindungen gebildet wurden, beispielsweise das dimere Aldolprodukt von Cyclopentanon oder Cyclopentanon-2-carbonsäurester.

### Verfahrensschritt (d)

Im Verfahrensschritt (d) des erfindungsgemäßen Verfahrens gemäß Ausführungsform I wird die in Verfahrensschritt (c) erhaltene wässrige Phase in eine zweite Kolonne K2 überführt. In dieser zweiten Kolonne erfolgt eine Trennung der wässrigen Phase in Wasser, ein Cyclopentanon/Wasser-Gemisch und gegebenenfalls noch vorhandene, in dem Verfahrensschritt (b) nicht abgetrennte und/oder zwischenzeitlich neu gebildete Leichtsieder. Anschließend werden die Leichtsieder über den Kopf der zweiten Kolonne und Wasser und gegebenenfalls vorhandene Hochsieder über den Sumpf der zweiten Kolonne ausgeschleust und ein Cyclopentanon/Wasser-Gemisch wird über einen Seitenabzug der zweiten Kolonne erhalten. Das Cyclopentanon/Wasser-Gemisch wird vorzugsweise in den Phasenscheider des Verfahrensschrittes (c) zurückgeführt.

Die zweite Kolonne K2 des Verfahrensschrittes (d) wird im Allgemeinen bei einem Druck von 650 bis 950 mbar, bevorzugt 700 bis 900 mbar, besonders bevorzugt 750 bis 850 mbar, betrieben. Die zweite Kolonne K2 wird im Allgemeinen bei einer Sumpftemperatur von 70 bis 100 °C, bevorzugt 75 bis 95 °C, besonders bevorzugt 80 bis 90 °C, betrieben.
Für die Destillation des Verfahrensschrittes (d) kommen übliche Apparaturen in Betracht, wie sie beispielsweise in Kirk-Othmer, Encyclopedia of Chemical Technology, 3. Ed., Vol. 7, John Wiley & Sons, New York, 1979, Seiten 870-881 beschrieben sind, beispielsweise Siebbodenkolonnen, Glockenbodenkolonnen, Packungskolonnen und Füllkörperkolonnen.

### Verfahrensschritt (e)

Die in Verfahrensschritt (c) erhaltene organische Phase, die Cyclopentanon, gegebenenfalls geringe Mengen Wasser und gegebenenfalls in dem Verfahrensschritt (b) nicht abgetrennte und/oder zwischenzeitlich neu gebildete Leicht- und/oder Hochsieder enthält, wird in eine dritte Kolonne K3 überführt, worin eine Trennung in gegebenenfalls noch vorhandene Leicht- und/oder Hochsieder, in ein Cyclopentanon/Wasser-Gemisch und hochreines Cyclopentanon erfolgt. Die Leichtsieder werden über den Kopf der dritten Kolonne ausgeschleust, während die Hochsieder über den Sumpf erhalten werden. Das ebenfalls über den Kopf der dritten Kolonne gewonnene Cyclopentanon/Wasser-Gemisch wird gegebenenfalls in den Phasenscheider des Verfahrensschrittes (c) zurückgeführt. Das Cyclopentanon wird am Seitenabzug der dritten Kolonne erhalten.

Die im Verfahrensschritt (e) verwendete dritte Kolonne wird bei einem Druck von 150 bis 450 mbar, bevorzugt 200 bis 400 mbar, besonders bevorzugt 250 bis 350 mbar, betrieben. Die dritte Kolonne K3 wird im Allgemeinen bei einer Sumpftemperatur von 80 bis 120 °C, bevorzugt 85 bis 115 °C, besonders bevorzugt 90 bis 110 °C, betrieben.

Für die Destillation kommen hierfür übliche Apparaturen in Betracht, wie sie beispielsweise in Kirk-Othmer, Encyclopedia of Chemical Technology, 3. Ed., Vol. 7, John Wiley & Sons, New York, 1979, Seiten 870-881 beschrieben sind, beispielsweise Siebbodenkolonnen, Glockenbodenkolonnen, Packungskolonnen und Füllkörperkolonnen.

### Ausführungsform II

### Verfahrensschritte (a) und (b)

Die Verfahrensschritte (a) und (b) des erfindungsgemäßen Verfahrens gemäß Ausführungsform II sind identisch zu den Verfahrensschritten (a) und (b) des erfindungsgemäßen Verfahrens gemäß Ausführungsform I. Daher wird auf obige Ausführungen verwiesen.

### Verfahrensschritt (c)

Das in Verfahrensschritt (b) erhaltene Cyclopentanon/Wasser-Gemisch wird in Verfahrensschritt (c) in eine wässrige Phase, die gegebenenfalls geringe Mengen Cyclopentanon enthält, und eine organische Phase, die Cyclopentanon und gegebenenfalls geringe Mengen an Wasser enthält, getrennt. Diese Trennung erfolgt in einem Phasenscheider und im Allgemeinen durch Kondensation auf Temperaturen, bei denen sich zwei Phasen bilden, beispielsweise 40 °C.

Die so erhaltene wässrige Phase wird bevorzugt in die erste Kolonne des Verfahrensschrittes (b) zurückgeführt. Sowohl die organische als auch die wässrige Phase können gegebenenfalls noch geringe Mengen an Hoch- und/oder Leichtsieder enthalten, die im Verfahrensschritt (b) nicht vollständig abgetrennt wurden oder sich zwischenzeitlich gebildet haben.

Die organische Phase enthält im Allgemeinen 75 bis 90 Gew.-%, bevorzugt 77,5 bis 87,5 Gew.-%, besonders bevorzugt 80 bis 85 Gew.-% Cyclopentanon, jeweils bezogen auf die organische Phase. Die wässrige Phase enthält im Allgemeinen 35 bis 15 Gew.-%, bevorzugt 32,5 bis 17,5 Gew.-%, besonders bevorzugt 30 bis 20 Gew.-%, Cyclopentanon, jeweils bezogen auf die wässrige Phase.

### Verfahrensschritt (d)

Die in Verfahrensschritt (c) erhaltene organische Phase, die Cyclopentanon enthält, wird in eine Trennwandkolonne überführt. In dieser Trennwandkolonne erfolgt eine Trennung der organischen Phase in gegebenenfalls noch vorhandene, in dem Verfahrensschritt (b) nicht abgetrennte und/oder zwischenzeitlich neu gebildete Leicht- und/oder Hochsieder, ein Cyclopentanon/Wasser-Gemisch und Cyclopentanon. Die Leichtsieder und das Cyclopentanon/Wasser-Gemisch werden über den Kopf der Trennwandkolonne und die Hochsieder über den Sumpf ausgeschleust, während das Cyclopentanon über einen Seitenabzug der Trennwandkolonne erhalten wird. Das Cyclopentanon/Wasser-Gemisch, das über den Kopf der Trennwandkolonne ausgeschleust wird, kann vorzugsweise in den Phasenscheider des Verfahrensschrittes (c) zurückgeführt werden.

Somit ersetzt die Trennwandkolonne des erfindungsgemäßen Verfahrens gemäß Ausführungsform II formal die beiden Kolonnen der Verfahrensschritte (d) und (e) des erfindungsgemäßen Verfahrens gemäß Ausführungsform I.
Die Funktionsweise von Trennwandkolonnen ist dem Fachmann bekannt. Trennwandkolonnen weisen typischerweise eine in Kolonnenlängsrichtung ausgerichtete Trennwand auf, die den Kolonneninnenraum in die folgenden Teilbereiche unterteilt: einen oberen gemeinsamen Kolonnenbereich, einen unteren gemeinsamen Kolonnenbereich sowie einen Zulaufteil und einen Entnahmeteil, jeweils mit Verstärkungsteil und Abtriebsteil. Das aufzutrennende Gemisch wird im Bereich des Zulaufsteils aufgegeben, eine Hochsiederfraktion wird aus dem Kolonnensumpf, eine Leichtsiederfraktion über den Kolonnenkopf und eine Mittelsiederfraktion aus dem Bereich des Entnahmeteils entnommen.

Bei der Trennung von Mebrstoffgemischen in eine Leichtsieder-, eine Mittelsieder- und eine Hochsiederfraktion werden üblicherweise Spezifikationen über den maximal zulässigen Anteil an Leichtsiedern und Hochsiedem in der Mittelsiederfraktion vorgegeben. Spezifiziert werden hierbei für das Trennproblem kritische Verbindungen, sogenannte Schlüsselkomponenten. Im vorliegenden Verfahren sind beispielsweise Wasser, Methanol (Leichtsieder) und Adipinsäurederivate (Hochsieder) Schlüsselkomponenten in der Cyclopentanon-Reindestillation.

Bezüglich der einsetzbaren trennwirksamen Einbauten in die Trennwandkolonnen gibt es grundsätzlich keine Einschränkungen. Hierzu sind sowohl Füllkörper als geordnete Packungen oder Böden geeignet. Bei den Packungskolonnen sind geordnete Blechpackungen mit einer spezifischen Oberfläche von im Allgemeinen 100 bis 500 m²/m³, bevorzugt 200 bis 400 m²/m³, besonders bevorzugt 250 bis 300 m²/m³, besonders geeignet.

Bei besonders hohen Anforderungen an die Produkte und insbesondere für den Fall, dass Packungen als trennwirksame Einbauten eingesetzt werden, wird die Trennwand im Allgemeinen mit einer thermischen Isolierung ausgestattet. Eine derartige Ausgestaltung der Trennwand ist beispielsweise in der EP-A 0 640 367 beschrieben, dessen Offenbarung durch Bezugnahme in die vorliegende Erfindung eingeschlossen ist.

Die Trennwandkolonne wird bei einem Betriebsdruck von im Allgemeinen 0,1 bis 1,0 bar, bevorzugt 0,3 bis 0,95 bar, besonders bevorzugt 0,5 bis 0,9 bar, betrieben. In einer bevorzugten Ausgestaltung der Trennwandkolonne können die Zulaufstelle des aufzutrennendes Stromes, d. h. der in Verfahrensschritt (c) erhaltenen organischen Phase, und die Entnahmestelle des Mittelsiederstromes, d. h. des hochreinen Cyclopentanons, auf unterschiedlicher Höhe in der Kolonne angeordnet sein.

In einer weiteren bevorzugten Verfahrensvariante kann der Rücklauf aus den oberen Kolonnenteilen so geregelt werden, dass das Verhältnis des Rücklaufstroms zum entnommenen Kopfstrom im Allgemeinen 0,5 bis 30, bevorzugt 1 bis 20, besonders bevorzugt 5 bis 10, beträgt. Als weiter vorteilhaft erwies sich ein Verhältnis von Trennblechhöhe zur Kolonnenhöhe von 1 zu 3, bevorzugt von 1 zu 1,5.

Gemäß einer weiteren bevorzugten Verfahrensvariante erfolgt die Entnahme des reinen Cyclopentanons über einen flüssigen oder gasförmigen Seitenabzug, welcher über den Sumpfstand geregelt wird.

In einer weiteren Variante des erfindungsgemäßen Verfahrens gemäß Ausführungsform II besteht die Möglichkeit, insbesondere im Falle des Auftretens von Leichtsiedern mit einem Siedepunkt zwischen dem Siedepunkt des Cyclopentanon/Wasser-Azeotrops und dem Siedepunkt von Cyclopentanon, diese Leicht- und/oder Mittelsieder über den Seitenabzug auszuschleusen und Cyclopentanon über den Sumpf zu gewinnen.

Alternativ ist es möglich, dass erfindungsgemäße Verfahren gemäß Ausführungsform I in Verfahrensschritt (e) mit einer Trennwandkolonne durchzuführen. Die Durchführung der Trennung in der Trennwandkolonne (Ausführungsform I) erfolgt dann analog dem Verfahrensschritt (d) des erfindungsgemäßen Verfahrens gemäß Ausführungsform II. In diesem Fall wird also nur die dritte Kolonne des erfindungsgemäßen Verfahrens gemäß Ausführungsform I durch eine Trennwandkolonne ersetzt, während die Verfahrensschritte (b) und (d) weiterhin mit herkömmlichen, oben beschriebenen Kolonnen durchgeführt werden.

Verunreinigungen des gemäß Ausführungsform I und II erhaltenen hochreinen Cyclopentanons werden - wenn überhaupt - in Form sehr geringer Anteile Methanol und Hochsieder (Adipinsäuredialkylester) nachgewiesen. Eventuell auftretende Hochsieder werden über den mengenmäßig sehr geringen Sumpfstrom ausgeschleust.

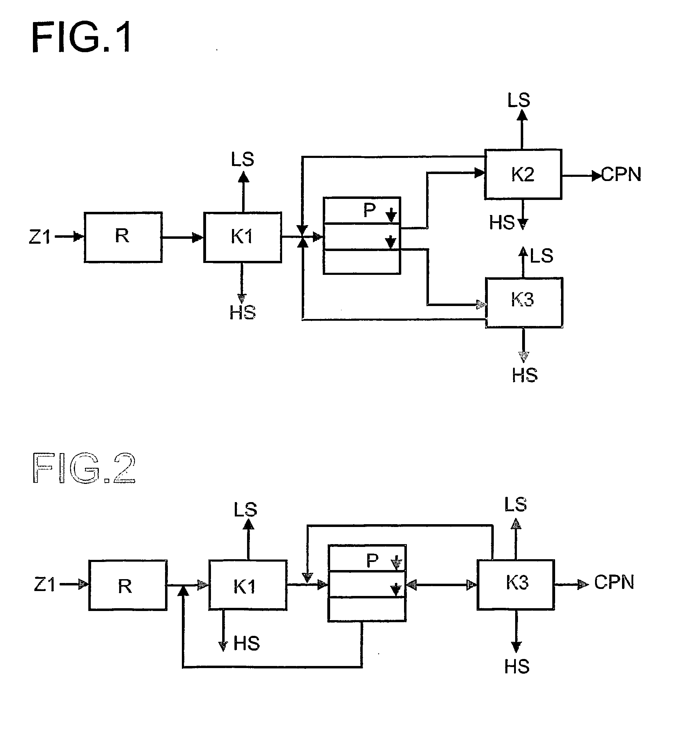

Eine besondere Ausgestaltung des erfindungsgemäßen Verfahrens gemäß Ausführungsform I ist in Fig. 1 dargestellt. In dieser Fig. bedeuten:
- R:: Reaktor
- K1:: Kolonne 1
- P:: Phasenscheider
- K2:: Kolonne 2
- K3:: Kolonne 3
- Z1:: Zufluss der Ausgangsverbindungen
- LS:: Leichtsieder
- HS:: Hochsieder
- CPN:: Cyclopentanon

In dem Reaktor (R) findet der Verfahrensschritt (a) statt. Die daraus resultierende Mischung wird in die Kolonne 1 (K1) überführt, worin in dieser besonderen Ausführungsform Leichtsieder (LS) über den Kopf und Hochsieder (HS) über den Sumpf abgetrennt werden. In dem anschließenden Phasenscheider (P) werden die organische und wässrige Phase getrennt. Die organische Phase wird anschließend in der Kolonne 2 (K2) und die wässrige Phase in der Kolonne 3 (K3) aufgearbeitet.

Eine besondere Ausgestaltung des erfindungsgemäßen Verfahrens gemäß Ausführungsform II ist in Fig. 2 dargestellt. In dieser Fig. bedeuten:
- R:: Reaktor
- K1:: Kolonne 1
- P:: Phasenscheider
- K3:: Trennwandkolonne
- Z1:: Zufluss der Ausgangsverbindungen
- LS:: Leichtsieder
- HS:: Hochsieder
- CPN:: Cyclopentanon

In dem Reaktor (R) findet der Verfahrensschritt (a) statt. Die daraus resultierende Mischung wird in die Kolonne 1 (K1) überführt, worin in dieser besonderen Ausführungsform Leichtsieder (LS) und Hochsieder (HS) abgetrennt werden. In dem anschließenden Phasenscheider (P) werden die organische und wässrige Phase abgetrennt. Die wässrige Phase wird in der Kolonne 1 (K1) zurückgeführt und die organische Phase wird in der Trennwandkolonne (TW) aufgearbeitet.

Weiterer Gegenstand der vorliegenden Erfindung ist die Verwendung von Alkalioxiden auf oxidischen Trägern zur Herstellung von Cyclopentanon ausgehend von mindestens einer Verbindung der allgemeinen Formel (I)

X-(CH₂)₄-COOR (I),

in der X einen Carbonsäure-, Carbonsäureester-, Formyl- oder Hydroxymethylrest darstellt und R Wasserstoff einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder ausgehend von mindestens einer Verbindung, die mit Wasser oder Alkoholen in mindestens eine Verbindung der allgemeinen Formel (I) übergehen kann.

Bezüglich Verbindungen, die mit Wasser und/oder Alkoholen in mindestens eine Verbindung der allgemeinen Formel (I) übergehen können, wird auf obige Ausführungen zum erfindungsgemäßen Verfahren verwiesen.

Bezüglich der zu verwendenden Katalysatoren wird ebenfalls auf die vorstehenden Ausführungen zum erfindungsgemäßen Verfahren verwiesen. Besonders geeignet sind Na₂O und K₂O auf Trägern, die ausgewählt sind aus der Gruppe bestehend aus Aluminiumoxiden, Siliziumdioxid, Titandioxid und deren Gemischen.

Diese Katalysatoren eignen sich im Allgemeinen für alle bisher genannten Ausgangsverbindungen, besonders für Carbonsäureester wie Adipinsäuremono- und Adipinsäurediester, Formylvaleriansäurester oder Hydroxycapronsäureester.

Die vorliegende Erfindung zeigt eine Reihe von Vorteilen gegenüber dem Stand der Technik:

Im Gegensatz zu den Herstellungsverfahren von Cyclopentanon durch Cyclisierung von Adipinsäure bzw. Adipinsäurederivaten des Standes der Technik erfolgt das erfindungsgemäße Verfahren mit hoher Ausbeute und einer hohen Standzeit des Cyclisierungskatalysators. Das dabei entstehende Cyclopentanon/Wasser-Gemisch lässt sich mit einem geringen apparativen und energetischen Aufwand zu Cyclopentanon mit einer hohen Reinheit aufarbeiten. Das durch das erfindungsgemäße Verfahren herstellbare Cyclopentanon weist im Allgemeinen eine Reinheit von mindestens 99 %, bevorzugt 99,5 %, besonders bevorzugt mindestens 99,9 % auf. Der Wassergehalt des erfindungsgemäßen Cyclopentanons ist im Allgemeinen kleiner als 2.500 ppm, bevorzugt kleiner als 2.000 ppm, besonders bevorzugt kleiner als 1.500 ppm, während die Farbzahl des Cyclopentanons im Allgemeinen weniger als 20 APHA, bevorzugt weniger als 15 APHA, besonders bevorzugt weniger als 10 APHA, beträgt. Die Aufarbeitung des Cyclopentanon/Wasser-Gemisches erfolgt dabei ohne einen zusätzlichen Aufreinigungsschritt wie Phasentrennung, Reinigung durch Kristallisation oder Vergrößerung der Mischungslücke. Darüber hinaus wird das Cyclopentanon in einem kontinuierlichen Verfahren gewonnen.
Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne das er dabei den Rahmen der vorliegenden Erfindung verlässt.

Die vorliegende Erfindung wird anhand der folgenden Ausführungsbeispiele veranschaulicht, welche die Erfindung jedoch keinesfalls beschränken.

### Ausführungsbeispiele

### Beispiel 1

### 1. Herstellung des Cyclisierungskatalysators

3 kg Al₂O₃ wurden mit 0,05 kg Kaliumcarbonat und 2,8 kg Wasser geknetet. Die gut durchmischte Masse wurde zu 4-mm-Strängen geformt und bei 120 °C getrocknet. Die getrockneten Stränge wurde bei 1200 °C calciniert.

### 2. Cyclisierung von Adipinsäuredimethylester

Die Cyclisierungsapparatur ist aus einem Verdampfer und einem Reaktor (34 x 4000 mm), aufgebaut, der 500 mm des nach 1.) hergestellten K₂O/Al₂O₃-Katalysators enthält.

Adipinsäuredimethylester und Wasser werden durch zwei Pumpen separat in den Verdampfer zudosiert, wo sie nach Verdampfung bei Reaktionstemperatur mit vorgeheiztem Stickstoff vermischt und dann zusammen in den Reaktor geleitet werden. Im Anschluss an den Reaktor wird der Reaktionsstrom in einem Kühler abgekühlt und das Kondensat in einem nachgeschalteten Abscheider von Abgasen abgetrennt.

Adipinsäuredimethylester wird gemäß EP-A 0 883 591 hergestellt und aus Stufe 4 des dort beschriebenen Verfahrens (siehe Ausführungsbeispiel) ausgeschleust. Durch fraktionierende Destillation wird er auf eine Reinheit von 95 bis 97% gebracht.

Die Zusammensetzung der Reaktionsausträge wird gaschromatographisch mit Diethylenglykoldimethylether als innerem Standart quantitativ analysiert.

Der Versuch wird bei 360°C/1 mbar, einer Katalysatorbelastung von 0,12 kg Adipinsäuredimethylester pro 1 Katalysator und pro Stunde unter Zuleiten von 5 mol Wasserdampf pro mol Adipinsäuredimethylester 500 Stunden lang in Rieselfahrweise durchgeführt. Pro Stunde werden außerdem 100 Liter Stickstoff zugeleitet.

Die gaschromatographisch ermittelte Cyclopentanon-Ausbeute während der gesamten Versuchszeit beträgt 93 bis 95%, der Esterumsatz ist größer 99%.

### 3. Destillative Cyclopentanon-Reinigung (zweistufig)

Aufgearbeitet wird der gemäß 2.) erhaltene Cyclisierungsaustrag.

Der Cyclisierungsaustrag wird einer Glockenbodenkolonne mit 50 theoretischen Trennstufen auf der Zulaufseite in Höhe des 38 theoretischen Bodens zudosiert.

Die Kolonne wird bei einem Kopfdruck von 900 mbar und einem Rücklaufverhältnis von 10 betrieben. Leichtsieder und Hochsieder werden ausgeschleust. Auf der Entnahmeseite der Kolonne wird auf der Höhe des 18. theoretischen Bodens das Cyclopentanon/Wasser-Gemisch, das zu 42,5 Gew.-% aus Cyclopentanon, zu 300 Gew.-ppm aus Methanol und 57,4 Gew.-% aus Wasser besteht, abgenommen. Das gasförmige Cyclopentanon/Wasser-Gemisch wird auf 40 °C abgekühlt und in einen Phasenscheider geleitet. Es trennt sich in eine wässrige und eine organische Phase auf. Die wässrige Phase wird in die Kolonne K1 zurückgeführt. Die organische Phase, die aus 84,8 Gew.-% Cyclopentanon, 15,2 Gew.-% Wasser und 200 Gew.-% ppm Methanol besteht, wird der Trennwandkolonne zugeführt.

Die Trennwandkolonne weist 84 theoretische Trennstufen auf und wird bei einem Betriebsdruck von 900 mbar und einem Rücklaufverhältnis von 10 betrieben. Die Dosierung des Feedstroms erfolgt auf der Zulaufseite der Kolonne in Höhe des 43. theoretischen Bodens und die Entnahme des reinen Cyclopentanons erfolgt auf der Höhe des 36. theoretischen Bodens auf der Entnahmeseite der Kolonne. Das unter diesen Bedingungen erzeugte Cyclopentanon weist eine Reinheit von 99,96 % auf. Als Nebenkomponenten wurden Methanol mit 10 ppm, Hochsieder (Adipinsäurediester) mit 70 ppm und Wasser mit 100 ppm Anteil detektiert. Die Farbzahl des Cyclopentanons beträgt weniger als 5 APHA. Leichtsieder werden über Kopf, Hochsieder über Sumpf ausgeschleust und das Cyclopentanon/Wasser-Gemisch wird in den Phasenscheider zurückgeführt.

## Patentansprüche

1. Kontinuierliches Verfahren zur Herstellung von Cyclopentanon, umfassend die folgenden Verfahrensschritte:
(a) Bereitstellung einer Mischung, die erhalten wird
durch Umsetzung
mindestens einer Verbindung der allgemeinen Formel (I)
X-(CH₂)₄-COOR (I),
in der X einen Carbonsäure-, Carbonsäureester-, Formyl- oder Hydroxymethylrest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder
durch Umsetzung
mindestens einer Verbindung, die mit Wasser und/oder Alkoholen in mindestens eine Verbindung der allgemeinen Formel (I) übergehen kann, an
mindestens einem Katalysator gegebenenfalls in Gegenwart von Wasser,
(b) Überführen der in Verfahrensschritt (a) erhaltenen Mischung enthaltend Cyclopentanon, Wasser und gegebenenfalls Hoch- und/oder Leichtsieder in eine erste Kolonne, worin eine Trennung der Mischung in ein Cyclopentanon/Wasser-Gemisch und gegebenenfalls vorhandene Hoch- und/oder Leichtsieder erfolgt, und Ausschleusen des Cyclopentanon/Wasser-Gemisches durch einen Seitenabzug der ersten Kolonne oder als Kopfprodukt der ersten Kolonne, wobei das Cyclopentanon/Wasser-Gemisch gegebenenfalls noch geringe Mengen an Hoch- und/oder Leichtsieder enthält,
(c) Trennung des in Verfahrensschritt (b) erhaltenen Cyclopentanon/Wasser-Gemisches in eine wässrige Phase enthaltend Wasser und gegebenenfalls geringe Mengen Cyclopentanon und eine organische Phase enthaltend Cyclopentanon und gegebenenfalls geringe Mengen Wasser in einem Phasenscheider, wobei die organische und/oder wässrige Phase gegebenenfalls noch geringe Mengen Hoch- und/oder Leichtsieder enthält,
(d) Überführen der in Verfahrensschritt (c) erhaltenen wässrigen Phase in eine zweite Kolonne, worin eine Trennung der wässrigen Phase in Wasser, ein Cyclopentanon/Wasser-Gemisch und gegebenenfalls noch vorhandene, in dem Verfahrensschritt (b) nicht abgetrennte und/oder zwischenzeitlich neu gebildete Hoch- und/oder Leichtsieder erfolgt, und Ausschleusen von Leichtsiedern über den Kopf der zweiten Kolonne, von Wasser und gegebenenfalls von Hochsiedem über den Sumpf der zweiten Kolonne und von einem Cyclopentanon/Wasser-Gemisch über einen Seitenabzug der zweiten Kolonne, wobei das Cyclopentanon/Wasser-Gemisch gegebenenfalls in den Phasenscheider des Verfahrensschrittes (c) zurückgeführt wird,
(e) Überführen der in Verfahrensschritt (c) erhaltenen organischen Phase enthaltend Cyclopentanon in eine dritte Kolonne, worin eine Trennung der organischen Phase in gegebenenfalls noch vorhandene, in dem Verfahrensschritt (b) nicht abgetrennte und/oder zwischenzeitlich neu gebildete Leicht- und/oder Hochsieder und Cyclopentanon erfolgt, Ausschleusen der über den Kopf der dritten Kolonne gewonnenen Leichtsieder sowie der über Sumpf gewonnenen Hochsieder, Ausschleusen und gegebenenfalls Zurückführen des ebenfalls über den Kopf der dritten Kolonne gewonnenen Cyclopentanon/Wasser-Gemisches in den Phasenscheider des Verfahrensschrittes (c) und Erhalt des Cyclopentanons im Seitenabzug der dritten Kolonne.

2. Kontinuierliches Verfahren zur Herstellung von Cyclopentanon, umfassend die folgenden Verfahrensschritte:
(a) Bereitstellung einer Mischung, die erhalten wird
durch Umsetzung
mindestens einer Verbindung der allgemeinen Formel (I)
X-(CH₂)₄-COOR (I),
in der X einen Carbonsäure-, Carbonsäureester-, Formyl- oder Hydroxymethylrest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder
durch Umsetzung
mindestens einer Verbindung, die mit Wasser und/oder Alkoholen in mindestens eine Verbindung der allgemeinen Formel (I) übergehen kann, an
mindestens einem Katalysator gegebenenfalls in Gegenwart von Wasser,
(b) Überführen der in Verfahrensschritt (a) erhaltenen Mischung enthaltend Cyclopentanon, Wasser und gegebenenfalls Hoch- und/oder Leichtsieder in eine erste Kolonne, worin eine Trennung der Mischung in ein Cyclopentanon/Wasser-Gemisch und gegebenenfalls vorhandene Hoch- und/oder Leichtsieder erfolgt, und Ausschleusen des Cyclopentanon/ Wasser-Gemisches durch einen Seitenabzug der ersten Kolonne bei Anwesenheit von Leichtsiedern oder als Kopfprodukt der ersten Kolonne bei Abwesenheit von Leichtsiedern, wobei das Cyclopentanon/Wasser-Gemisch gegebenenfalls noch geringe Mengen Hoch- und/oder Leichtsieder enthält,
(c) Trennung des in Verfahrensschritt (b) erhaltenen Cyclopentanon/Wasser-Gemisches in eine wässrige Phase enthaltend Wasser und gegebenenfalls geringe Mengen Cyclopentanon und eine organische Phase enthaltend Cyclopentanon und gegebenenfalls geringe Mengen Wasser in einem Phasenscheider, wobei die wässrige Phase gegebenenfalls in die erste Kolonne des Verfahrensschrittes (b) zurückgeführt wird und die organische und/oder wässrige Phase gegebenenfalls noch geringe Mengen Hoch- und/oder Leichtsieder enthält,
(d) Überführen der in Verfahrensschritt (c) erhaltenen organischen Phase in eine Trennwandkolonne, worin eine Trennung der organischen Phase in gegebenenfalls noch vorhandene, in dem Verfahrensschritt (b) nicht abgetrennte und/oder zwischenzeitlich neu gebildete Leicht- und/oder Hochsieder, ein Cyclopentanon/Wasser-Gemisch und Cyclopentanon erfolgt, und Ausschleusen von Leichtsiedern und dem Cyclopentanon/Wasser-Gemisch über den Kopf der Trennwandkolonne, von Hochsiedern über den Sumpf der Trennwandkolonne und von Cyclopentanon über einen Seitenabzug der Trennwandkolonne, wobei das Cyclopentanon/Wasser-Gemisch gegebenenfalls in den Phasenscheider des Verfahrensschrittes (c) zurückgeführt wird.

3. Verfahren nach Anspruch 1, **dadurch gekennzeichnet, dass** anstelle der dritten Kolonne in Verfahrensschritt (e) eine Trennwandkolonne verwendet wird.

4. Verfahren nach einem der Ansprüche 1 bis 3, **dadurch gekennzeichnet, dass** die Umsetzung in Verfahrensschritt (a) bei Temperaturen von 200 bis 400 °C, bevorzugt 210 bis 350 °C, besonders bevorzugt 220 bis 330 °C, durchgeführt wird.

5. Verfahren nach einem der Ansprüche 1 bis 4, **dadurch gekennzeichnet, dass** die Katalysatorbelastung in Verfahrensschritt (a) von 0,1 bis 4 kg wasserfreie Verbindung der allgemeinen Formel (I) pro kg Katalysator und pro Stunde, bevorzugt 0,1 bis 2 kg wasserfreie Verbindung der allgemeinen Formel (I) pro kg Katalysator und pro Stunde, besonders bevorzugt 0,1 bis 1 kg wasserfreie Verbindung der allgemeinen Formel (I) pro kg Katalysator und pro Stunde, beträgt.

6. Verfahren nach einem der Ansprüche 1 bis 5, **dadurch gekennzeichnet, dass** in Verfahrensschritt (a) als Katalysatoren Metalle, Metalloxide, Metallhydroxide, Metallsalze und/oder Metall-Doppelsalze von Elementen der I. bis XIV. Hauptgruppe des Periodensystems und/oder Seltenerdmetallen, bevorzugt Alkalioxide und/oder Seltenerdmetalle auf oxidischen Trägem, verwendet werden.

7. Verfahren nach Anspruch 6, **dadurch gekennzeichnet, dass** als Katalysator 0,01 bis 5 Gew.-%, bevorzugt 0,1 bis 3 Gew.-%, besonders bevorzugt 0,3 bis 2 Gew.-%, jeweils bezogen auf den gesamten Katalysator, Alkalioxid auf oxidischen Trägern verwendet wird.

8. Verfahren nach Anspruch 6 oder 7, **dadurch gekennzeichnet, dass** als katalytisch aktives Material des Katalysators Natrium- und/oder Kaliumoxid verwendet wird und/oder dass das Trägermaterial des Katalysators ausgewählt ist aus der Gruppe bestehend aus Aluminiumoxiden, Siliziumoxid, Titandioxid und deren Gemischen.

9. Verfahren nach einem der Ansprüche 1 bis 8, **dadurch gekennzeichnet, dass** die in Verfahrensschritt (b) verwendete erste Kolonne 40 bis 80 theoretische Böden, bevorzugt 50 bis 70 theoretische Böden, besonders bevorzugt 55 bis 65 theoretische Böden, aufweist und/oder die Kolonne bei einer Sumpftemperatur von 70 bis 120 °C, bevorzugt 80 bis 110 °C, besonders bevorzugt 90 bis 100 °C, und/oder bei einem Druck von 800 bis 1000 mbar, bevorzugt 850 bis 950 mbar, besonders bevorzugt 875 bis 925 mbar, betrieben wird.

10. Verfahren nach einem der Ansprüche 1 und 3 bis 9, **dadurch gekennzeichnet, dass** die in Verfahrensschritt (d) verwendete zweite Kolonne bei einem Druck von 650 bis 950 mbar, bevorzugt 700 bis 900 mbar, besonders bevorzugt 750 bis 850 mbar, betrieben wird und/oder die Kolonne bei einer Sumpftemperatur von 70 bis 100 °C, bevorzugt 75 bis 95 °C, besonders bevorzugt 80 bis 90°C, betrieben wird.

11. Verfahren nach einem der Ansprüche 1 und 4 bis 10, **dadurch gekennzeichnet, dass** die in Verfahrensschritt (e) verwendete dritte Kolonne bei einem Druck von 150 bis 450 mbar, bevorzugt 200 bis 400 mbar, besonders bevorzugt 250 bis 350 mbar, betrieben wird und/oder die Kolonne bei einer Sumpftemperatur von 80 bis 120 °C, bevorzugt 85 bis 115 °C, besonders bevorzugt 90 bis 110°C, betrieben wird.

12. Verfahren nach einem der Ansprüche 2 bis 11, **dadurch gekennzeichnet, dass** als trennwirksame Einbauten in der Trennwandkolonne Füllkörper als geordnete Packungen verwendet werden, wobei die Packungen aus geordneten Blechpackungen mit einer spezifischen Oberfläche von 100 bis 500 m²/m³, bevorzugt 200 bis 400 m²/m³, besonders bevorzugt 250 bis 300 m²/m³, bestehen.

13. Verfahren nach einem der Ansprüche 2 bis 12, **dadurch gekennzeichnet, dass** die Trennwandkolonne bei einem Betriebsdruck von 0,1 bis 1,0 bar, bevorzugt 0,3 bis 0,95 bar, besonders bevorzugt 0,5 bis 0,9 bar, betrieben wird.

14. Verwendung von Natrium- und/oder Kaliumoxid auf oxidischen Trägem, wobei das Trägermaterial ausgewählt ist aus der Gruppe bestehend aus Aluminiumoxiden, Siliziumdioxid und deren Gemischen, zur Herstellung von Cyclopentanon ausgehend von mindestens einer Verbindung der allgemeinen Formel (I)
X-(CH₂)₄-COOR (I),
in der X einen Carbonsäure-, Carbonsäureester-, Formyl- oder Hydroxymethylrest darstellt und R Wasserstoff, einen aliphatischen Rest mit 1 bis 6 Kohlenstoffatomen, einen cycloaliphatischen, araliphatischen oder aromatischen Rest mit 6 bis 12 Kohlenstoffatomen darstellt, und/oder ausgehend von mindestens einer Verbindung, die mit Wasser
oder Alkoholen in mindestens eine Verbindung der allgemeinen Formel I übergehen kann.

15. Verwendung nach Anspruch 14, **dadurch gekennzeichnet, dass** man 0,01 bis 5 Gew.-%, bevorzugt 0,1 bis 3 Gew.-%, besonders bevorzugt 0,3 bis 2 Gew.-%, jeweils bezogen auf den gesamten Katalysator, Alkalioxid auf oxidischen Trägern als Katalysatoren verwendet.

## Claims

1. A continuous process for the preparation of cyclopentanone comprising the following process steps:
(a) provision of a mixture which is obtained
by reaction
of at least one compound of the formula (I)
X-(CH₂)₄-COOR (I),
in which X is a carboxylic acid, carboxylic ester, formyl or hydroxymethyl radical, and R is hydrogen, an aliphatic radical having 1 to 6 carbon atoms, a cycloaliphatic, araliphatic or aromatic radical having 6 to 12 carbon atoms, and/or
by reaction
of at least one compound which, with water and/or alcohols, can convert to at least one compound of the formula (I),
over
at least one catalyst, optionally in the presence of water,
(b) transferring the mixture obtained in process step (a) and comprising cyclopentanone, water and if appropriate high-boiling and/or low-boiling components to a first column in which separation of the mixture takes place into a cyclopentanone/water mixture and high-boiling and/or low-boiling components which are, if appropriate, present, and removing the cyclopentanone/water mixture through a side take-off of the first column or as top product of the first column, where the cyclopentanone/water mixture, if appropriate, also comprises small amounts of high-boiling and/or low-boiling components,
(c) separation of the cyclopentanone/water mixture obtained in process step (b) into an aqueous phase comprising water and if appropriate small amounts of cyclopentanone and an organic phase comprising cyclopentanone and if appropriate small amounts of water in a phase separator, where the organic phase and/or aqueous phase, if appropriate, also comprises small amounts of high-boiling and/or low-boiling components,
(d) transferring the aqueous phase obtained in process step (c) to a second column in which separation of the aqueous phase takes place into water, a cyclopentanone/water mixture and high-boiling and/or low-boiling components which are, if appropriate, still present, were not separated off in process step (b) and/or have newly formed in the meantime, and removal of low-boiling components via the top of the second column, of water and if appropriate of high-boiling components via the bottom of the second column and of a cyclopentanone/water mixture via a side take-off of the second column, while the cyclopentanone/water mixture is, if appropriate, returned to the phase separator of process step (c),
(e) transferring the organic phase obtained in process step (c) and comprising cyclopentanone to a third column in which separation of the organic phase takes place into low-boiling and/or high-boiling components which are, if appropriate, still present, were not separated off in process step (b) and/or have newly formed in the meantime and cyclopentanone, removal of the low-boiling components obtained via the top of the third column, and of the high-boiling components obtained via the bottom, removal and, if appropriate, return of the cyclopentanone/water mixture likewise obtained via the top of the third column to the phase separator of process step (c) and obtaining the cyclopentanone in the side take-off of the third column.

2. A continuous process for the preparation of cyclopentanone comprising the following process steps:
(a) provision of a mixture which is obtained
by reaction
of at least one compound of the formula (I)
X-(CH₂)₄-COOR (I),
in which X is a carboxylic acid, carboxylic ester, formyl or hydroxymethyl radical, and R is hydrogen, an aliphatic radical having 1 to 6 carbon atoms, a cycloaliphatic, araliphatic or aromatic radical having 6 to 12 carbon atoms, and/or
by reaction
of at least one compound which, with water and/or alcohols, can convert to at least one compound of the formula (I),
over
at least one catalyst, if appropriate in the presence of water,
(b) transferring the mixture obtained in process step (a) and comprising cyclopentanone, water and if appropriate high-boiling and/or low-boiling components to a first column in which separation of the mixture takes place into a cyclopentanone/water mixture and high-boiling and/or low-boiling components which are, if appropriate, present, and removing the cyclopentanone/water mixture through a side take-off of the first column if low-boiling components are present, or as top product of the first column if low-boiling components are absent, where the cyclopentanone/water mixture, if appropriate, also comprises small amounts of high-boiling and/or low-boiling components,
(c) separation of the cyclopentanone/water mixture obtained in process step (b) into an aqueous phase comprising water and if appropriate small amounts of cyclopentanone and an organic phase comprising cyclopentanone and if appropriate small amounts of water in a phase separator, where the aqueous phase is, if appropriate, returned to the first column of process step (b), and the organic and/or aqueous phase, if appropriate, also comprises small amounts of high-boiling and/or low-boiling components,
(d) transferring the organic phase obtained in process step (c) to a dividing-wall column in which separation of the organic phase takes place into low-boiling and/or high-boiling components which are, if appropriate, still present, were not separated off in process step (b) and/or have newly formed in the meantime, a cyclopentanone/water mixture and cyclopentanone, and removal of low-boiling components and the cyclopentanone/water mixture via the top of the dividing-wall column, of high-boiling components via the bottom of the dividing-wall column and of cyclopentanone via a side take-off of the dividing-wall column, where the cyclopentanone/water mixture is, if appropriate, returned to the phase separator of process step (c).

3. The process according to claim 1, wherein a dividing-wall column is used instead of the third column in process step (e).

4. The process according to any of claims 1 to 3, wherein the reaction in process step (a) is carried out at temperatures of from 200 to 400°C, preferably 210 to 350°C, particularly preferably 220 to 330°C.

5. The process according to any of claims 1 to 4, wherein the catalyst space velocity in process step (a) is from 0.1 to 4 kg of anhydrous compound of the formula (1) per kg of catalyst and per hour, preferably 0.1 to 2 kg of anhydrous compound of the formula (I) per kg of catalyst and per hour, particularly preferably 0.1 to 1 kg of anhydrous compound of the formula (I) per kg of catalyst and per hour.

6. The process according to any of claims 1 to 5, wherein the catalysts used in process step (a) are metals, metal oxides, metal hydroxides, metal salts and/or metal double salts of elements of main group I to XIV of the Periodic Table of the Elements and/or rare earth metals, preferably alkali metal oxides and/or rare earth metals, on oxidic supports.

7. The process according to claim 6, wherein the catalyst used is 0.01 to 5% by weight, preferably 0.1 to 3% by weight, particularly preferably 0.3 to 2% by weight, in each case based on the total catalyst, of alkali metal oxide on oxidic supports.

8. The process according to claim 6 or 7, wherein the catalytically active material of the catalyst used is sodium oxide and/or potassium oxide and/or the support material of the catalyst is chosen from the group consisting of aluminum oxides, silicon oxide, titanium dioxide and mixtures thereof.

9. The process according to any of claims 1 to 8, wherein the first column used in process step (b) has 40 to 80 theoretical plates, preferably 50 to 70 theoretical plates, particularly preferably 55 to 65 theoretical plates, and/or the column is operated at a bottoms temperature of from 70 to 120°C, preferably 80 to 110°C, particularly preferably 90 to 100°C, and/or at a pressure of from 800 to 1 000 mbar, preferably 850 to 950 mbar, particularly preferably 875 to 925 mbar.

10. The process according to any of claims 1 and 3 to 9, wherein the second column used in process step (d) is operated at a pressure of from 650 to 950 mbar, preferably 700 to 900 mbar, particularly preferably 750 to 850 mbar, and/or the column is operated at a bottoms temperature of from 70 to 100°C, preferably 75 to 95°C, particularly preferably 80 to 90°C.

11. The process according to any of claims 1 and 4 to 10, wherein the third column used in process step (e) is operated at a pressure of from 150 to 450 mbar, preferably 200 to 400 mbar, particularly preferably 250 to 350 mbar, and/or the column is operated at a bottoms temperature of from 80 to 120°C, preferably 85 to 115°C, particularly preferably 90 to 110°C.

12. The process according to any of claims 2 to 11, wherein the separation-effective internals in the dividing-wall column are dumped packings as arranged packings, where the packings consist of arranged sheet-metal packings with a specific surface area of from 100 to 500 m²/m³, preferably 200 to 400 m²/m³, particularly preferably 250 to 300 m²/m³.

13. The process according to any of claims 2 to 12, wherein the dividing-wall column is operated at an operating pressure of from 0.1 to 1.0 bar, preferably 0.3 to 0.96 bar, particularly preferably 0.5 to 0.9 bar.

14. The use of sodium oxide and/or potassium oxide on oxidic supports, the support material thereof being chosen from the group consisting of aluminum oxides, silicon dioxide and mixtures thereof for the preparation of cyclopentanone starting from at least one compound of the formula (I)
X-(CH₂)₄-COOR (I),
in which X is a carboxylic acid, carboxylic ester, formyl or hydroxymethyl radical, and R is hydrogen, an aliphatic radical having 1 to 6 carbon atoms, a cycloaliphatic, araliphatic or aromatic radical having 6 to 12 carbon atoms, and/or starting from at least one compound which, with water or alcohols, can convert to at least one compound of the formula 1.

15. The use according to claim 14, wherein 0.01 to 5% by weight, preferably 0.1 to 3% by weight, particularly preferably 0.3 to 2% by weight, in each case based on the total catalyst, of alkali metal oxide on oxidic supports are used as catalysts.

## Revendications

1. Procédé de préparation en continu de cyclopentanone, comprenant les étapes suivantes :
(a) une préparation d'un mélange qui est obtenu par réaction
d'au moins un composé de la formule générale (I) :
X - (CH₂) ₄ - COOR (I)
dans laquelle X représente un radical d'acide carboxylique, d'ester d'acide carboxylique, formyle ou hydroxyméthyle et R représente de l'hydrogène, un radical aliphatique comportant 1 à 6 atomes de carbone, un radical cycloaliphatique, araliphatique ou aromatique comportant 6 à 12 atomes de carbone, et/ou
par réaction
d'au moins un composé qui peut se transformer avec de l'eau et/ou des alcools en au moins un composé de la formule générale (I),
sur
au moins un catalyseur, éventuellement en présence d'eau,
(b)une conversion du mélange obtenu dans l'étape (a) du procédé et contenant de la cyclopentanone, de l'eau et éventuellement des substances à haut et/ou bas point d'ébullition dans une première colonne, dans laquelle a lieu une séparation du mélange en un mélange de cyclopentanone/eau et en substances à haut et/ou bas point d'ébullition éventuellement présentes, et une évacuation par éclusage du mélange de cyclopentanone/eau par un soutirage latéral de la première colonne ou sous la forme de produit de tête de la première colonne, le mélange de cyclopentanone/eau contenant éventuellement encore de faibles quantités de substances à haut et/ou bas point d'ébullition,
(c)une séparation du mélange de cyclopentanone/eau obtenu dans l'étape (b) du procédé en une phase aqueuse contenant de l'eau et éventuellement de faibles quantités de cyclopentanone et en une phase organique contenant de la cyclopentanone et éventuellement de faibles quantités d'eau dans un séparateur de phases, la phase organique et/ou la phase aqueuse contenant éventuellement encore de faibles quantités de substances à haut et/ou bas point d'ébullition,
(d)une conversion de la phase aqueuse obtenue dans l'étape (c) du procédé dans une deuxième colonne, dans laquelle a lieu une séparation de la phase aqueuse en eau, en un mélange de cyclopentanone/eau et en substances à haut et/ou bas point d'ébullition éventuellement encore présentes, non isolées dans l'étape (b) du procédé et/ou nouvellement formées dans l'intervalle, et une évacuation par éclusage des substances à bas point d'ébullition par la tête de la deuxième colonne, d'eau et éventuellement de substances à haut point d'ébullition par le fond de la deuxième colonne et d'un mélange de cyclopentanone/eau par un soutirage latéral de la deuxième colonne, le mélange de cyclopentanone/eau étant éventuellement recyclé dans le séparateur de phases de l'étape (c) du procédé,
(e)une conversion de la phase organique obtenue dans l'étape (c) du procédé et contenant de la cyclopentanone dans une troisième colonne, dans laquelle a lieu une séparation de la phase organique en substances à bas et/ou haut point d'ébullition éventuellement encore présentes, non isolées dans l'étape (b) du procédé et/ou nouvellement formées dans l'intervalle et en cyclopentanone, une évacuation par éclusage des substances à bas point d'ébullition obtenues par la tête de la troisième colonne ainsi que des substances à haut point d'ébullition obtenues par le fond, une évacuation par éclusage et éventuellement un recyclage du mélange cyclopentanone/eau également obtenu par la tête de la troisième colonne dans le séparateur de phases de l'étape (c) du procédé et une obtention de la cyclopentanone dans le soutirage latéral de la troisième colonne.

2. Procédé de préparation en continu de cyclopentanone, comprenant les étapes de procédé suivantes :
(a) une préparation d'un mélange qui est obtenu
par réaction
d'au moins un composé de la formule générale (I) :
X - (CH₂) ₄ - COOR (I)
dans laquelle X représente un radical d'acide carboxylique, d'ester d'acide carboxylique, formyle ou hydroxyméthyle et R représente de l'hydrogène, un radical aliphatique comportant 1 à 6 atomes de carbone, un radical cycloaliphatique, araliphatique ou aromatique comportant 6 à 12 atomes de carbone, et/ou
par réaction
d'au moins un composé qui peut se transformer avec de l'eau et/ou des alcools en au moins un composé de la formule générale (I),
sur
au moins un catalyseur, éventuellement en présence d'eau,
(b)une conversion du mélange obtenu dans l'étape (a) du procédé et contenant de la cyclopentanone, de l'eau et éventuellement des substances à haut et/ou bas point d'ébullition dans une première colonne, dans laquelle a lieu une séparation du mélange en un mélange de cyclopentanone/eau et en substances à haut et/ou bas point d'ébullition éventuellement présentes, et une évacuation par éclusage du mélange de cyclopentanone/eau par un soutirage latéral de la première colonne lors de la présence de substances à bas point d'ébullition ou sous la forme de produit de tête de la première colonne en l'absence de substances à bas point d'ébullition, le mélange de cyclopentanone/eau contenant éventuellement encore de faibles quantités de substances à haut et/ou bas point d'ébullition,
(c)une séparation du mélange de cyclopentanone/eau obtenu dans l'étape (b) du procédé en une phase aqueuse contenant de l'eau et éventuellement de faibles quantités de cyclopentanone et en une phase organique contenant de la cyclopentanone et éventuellement de faibles quantités d'eau dans un séparateur de phases, la phase aqueuse étant éventuellement recyclée dans la première colonne de l'étape (b) du procédé et la phase organique et/ou aqueuse contenant éventuellement encore de faibles quantités de substances à haut et/ou bas point d'ébullition,
(d)une conversion de la phase organique obtenue dans l'étape (c) du procédé dans une colonne à paroi séparatrice, dans laquelle a lieu une séparation de la phase organique en substances à bas et/ou haut point d'ébullition éventuellement encore présentes, non isolées dans l'étape (b) du procédé et/ou nouvellement formées dans l'intervalle, en un mélange de cyclopentanone/eau et en cyclopentanone, et une évacuation par éclusage des substances à bas point d'ébullition et du mélange de cyclopentanone/eau par la tête de la colonne à paroi séparatrice, des substances à haut point d'ébullition par le fond de la colonne à paroi séparatrice et de la cyclopentanone par l'intermédiaire d'un soutirage latéral de la colonne à paroi séparatrice, le mélange de cyclopentanone/eau étant éventuellement recyclé dans le séparateur de phases de l'étape (c) du procédé.

3. Procédé suivant la revendication 1, **caractérisé en ce que**, au lieu de la troisième colonne, on utilise dans l'étape (e) du procédé une colonne à paroi séparatrice.

4. Procédé suivant l'une des revendications 1 à 3, **caractérisé en ce que** la réaction dans l'étape (a) du procédé est effectuée à des températures de 200 à 400°C, avantageusement de 210 à 350°C, particulièrement avantageusement de 220 à 330°C.

5. Procédé suivant l'une des revendications 1 à 4, **caractérisé en ce que** la sollicitation du catalyseur dans l'étape (a) du procédé est de 0,1 à 4 kg de composé anhydre de la formule générale (I) par kg de catalyseur et par heure, avantageusement de 0,1 à 2 kg de composé anhydre de la formule générale (I) par kg de catalyseur et par heure, particulièrement avantageusement de 0,1 à 1 kg de composé anhydre de la formule générale (I) par kg de catalyseur et par heure.

6. Procédé suivant l'une des revendications 1 à 5, **caractérisé en ce que**, dans l'étape (a) du procédé, on utilise comme catalyseurs des métaux, des oxydes métalliques, des hydroxydes métalliques, des sels métalliques et/ou des sels doubles métalliques des éléments du groupe principal I à XIV du système périodique et/ou des métaux des terres rares, avantageusement des oxydes de métal alcalin et/ou des métaux des terres rares sur des supports oxydés.

7. Procédé suivant la revendication 6, **caractérisé en ce que**, comme catalyseur, on utilise 0,01 à 5 % en poids, avantageusement 0,1 à 3 % en poids, particulièrement avantageusement 0,3 à 2 % en poids, respectivement par rapport au catalyseur total, d'oxyde de métal alcalin sur des supports oxydés.

8. Procédé suivant la revendication 6 ou 7, **caractérisé en ce que**, comme matière catalytiquement active du catalyseur, on utilise de l'oxyde de sodium et/ou de potassium et/ou **en ce que** la matière de support du catalyseur est choisie parmi le groupe constitué d'oxydes d'aluminium, d'oxyde de silicium, de dioxyde de titane et de leurs mélanges.

9. Procédé suivant l'une des revendications 1 à 8, **caractérisé en ce que** la première colonne utilisée dans l'étape (b) du procédé présente 40 à 80 plateaux théoriques, avantageusement 50 à 70 plateaux théoriques, particulièrement avantageusement 55 à 65 plateaux théoriques et/ou la colonne est mise en service à une température de fond de 70 à 120°C, avantageusement de 80 à 110°C, particulièrement avantageusement de 90 à 100°C, et/ou à une pression de 800 à 1000 mbars, avantageusement de 850 à 950 mbars, particulièrement avantageusement de 875 à 925 mbars.

10. Procédé suivant l'une des revendications 1 et 3 à 9, **caractérisé en ce que** la deuxième colonne utilisée dans l'étape (d) du procédé est mise en service à une pression de 650 à 950 mbars, avantageusement de 700 à 900 mbars, particulièrement avantageusement de 750 à 850 mbars et/ou la colonne est mise en service à une température de fond de 70 à 100°C, avantageusement de 75 à 95°C, particulièrement avantageusement de 80 à 90°C.

11. Procédé suivant l'une des revendications 1 et 4 à 10, **caractérisé en ce que** la troisième colonne utilisée dans l'étape (e) du procédé est mise en service à une pression de 150 à 450 mbars, avantageusement de 200 à 400 mbars, particulièrement avantageusement de 250 à 350 mbars et/ou la colonne est mise en service à une température de fond de 80 à 120°C, avantageusement de 85 à 115°C, particulièrement avantageusement de 90 à 110°C.

12. Procédé suivant l'une des revendications 2 à 11, **caractérisé en ce que**, comme éléments incorporés à action de séparation dans la colonne à paroi séparatrice, on utilise des corps de remplissage sous la forme de garnissages ordonnés, les garnissages étant constitués de garnissages en tôle ordonnés ayant une surface spécifique de 100 à 500 m²/m³, avantageusement de 200 à 400 m²/m³ , particulièrement avantageusement de 250 à 300 m²/m³.

13. Procédé suivant l'une des revendications 2 à 12, **caractérisé en ce que** la colonne à paroi séparatrice est mise en service à une pression de 0,1 à 1,0 bar, avantageusement de 0,3 à 0, 95 bar, particulièrement avantageusement de 0,5 à 0,9 bar.

14. Utilisation d'oxyde de sodium et/ou de potassium sur des supports oxydés, la matière de support étant choisie parmi le groupe constitué des oxydes d'aluminium, du dioxyde de silicium et de leurs mélanges, pour la préparation de cyclopentanone à partir d'au moins un composé de la formule générale (I) :
X - (CH₂)₄ - COOR (I)
dans laquelle X représente un radical d'acide carboxylique, d'ester d'acide carboxylique, formyle ou hydroxyméthyle et R représente de l'hydrogène, un radical aliphatique comportant 1 à 6 atomes de carbone, un radical cycloaliphatique, araliphatique ou aromatique comportant 6 à 12 atomes de carbone, et/ou en partant d'au moins un composé qui, avec de l'eau ou des alcools, peut se transformer en au moins un composé de la formule générale (I).

15. Utilisation suivant la revendication 14, **caractérisée en ce que**, comme catalyseurs, on utilise 0,01 à 5 % en poids, avantageusement 0,1 à 3 % en poids, particulièrement avantageusement 0,3 à 2 % en poids, respectivement par rapport au catalyseur total, d'oxyde de métal alcalin sur des supports oxydés.
